# EUROPEAN PATENT SPECIFICATION

(11) **EP 0 744 891 B1**
(45) Date of publication and mention of the grant of the patent: **03.10.2007**
(21) Application number: 95911012.3
(22) Date of filing: 17.02.1995
(51) Int. Cl.: A01K 67/027, C12N 15/63

(54) **TRANSGENIC FIBRINOGEN**
TRANSGENES FIBRINOGEN
FIBRINOGENE TRANSGENIQUE

(30) Priority: 18.02.1994 US 198068
(43) Date of publication of application: 04.12.1996
(73) Proprietor: The American National Red Cross, Washington, DC 20006 (US); VIRGINIA TECH INTELLECTUAL PROPERTIES, INC., Blacksburg, VA 24060 (US); THE UNIVERSITY OF NORTH CAROLINA AT CHAPEL HILL, Chapel Hill, NC 27599-4100 (US)
(72) Inventor: VELANDER, William, H., Blacksburg, VA 24060 (US); LORD, Susan, T., Chapel Hill, NC 27599 (US); DROHAN, William, N., Springfield, VA 22152 (US); LUBON, Henryk, Rockville, MD 20855 (US); JOHNSON, John, L., Blacksburg, VA 24060 (US); RUSSELL, Christopher, G., Blacksburg, VA 24060 (US)
(74) Representative: Gardner, Rebecca Katherine
(86) International application number: PCT/US1995/001944
(87) International publication number: WO 1995/022249

(56) References cited:
- WO-A-92/11757
- FIBRINOLYSIS, vol. 8, no. 1, 1994 page 102 D. PRUNKARD ET AL. 'Expression of recombinant human fibrinogen in the milk of transgenic mice'
- JOURNAL OF CONTROLLED RELEASE, vol. 29, 1994 pages 213-221, S.H. LEE ET AL. 'Production of biomedical proteins in the milk of transgenic dairy cows: the state of the art'
- BLOOD COAGULATION AND FIBRINOLYSIS, vol. 4, 1993 pages 55-59, S.T. LORD ET AL. 'Purification and characterization of recombinant human fibrinogen'
- JOURNAL OF BIOLOGICAL CHEMISTRY, vol. 266, no. 8, 15 March 1991 pages 4758-4763, S. N. ROY ET AL. 'Assembly and secretion of recombinant human fibrinogen'
- BIOCHEMISTRY, vol. 22, 1983 pages 3237-3244, M.W. RIXON ET AL. 'Characterization of a complementary DNA coding for the alfa chain of human fibrinogen'
- BIOCHEMISTRY, vol. 22, 1983 pages 3250-3256, D.W. CHUNG ET AL. 'Characterization of a cDNA coding for the gamma chain of human fibrinogen'
- BIOCHEMISTRY, vol. 22, 1983 pages 3244-3250, D.W. CHUNG ET AL. 'Characterisation of a cDNA coding for the beta chain of human fibrinogen'

### SEQUENCE LISTING

(1) GENERAL INFORMATION:
   (i) APPLICANT:
      (A) ADDRESSEE: American National Red Cross
      (B) STREET: 1730 E Street, N.W.
      (C) CITY: Washington, D.C.
      (E) COUNTRY: USA
      (F) POSTAL CODE: 20006
   (i) APPLICANT:
      (A) ADDRESSEE: Virginia Tech Intellectual Properties, Inc.
      (B) STREET: 1900 Kraft Drive
      (C) CITY: Blacksburg
      (D) STATE OR PROVINCE: Virginia
      (E) COUNTRY: USA
      (F) POSTAL CODE: 24060
   (i) APPLICANT:
      (A) ADDRESSEE: University of North Carolina, Office of Research Services
      (B) STREET: Campus Box 4100
      (C) CITY: Chapel Hill
      (D) STATE OR PROVINCE: North Carolina
      (E) COUNTRY: USA
      (F) POSTAL CODE: 27599
   (ii) TITLE OF INVENTION: TRANSGENIC FIBRINOGEN
   (iii) NUMBER OF SEQUENCES: 12
   (iv) COMPUTER READABLE FORM:
      (A) MEDIUM TYPE: Floppy disk
      (B) COMPUTER: IBM PC compatible
      (C) OPERATING SYSTEM: PC-DOS/XS-DOS
      (D) SOFTWARE: PatentIn Release #1.0, Version #1.25
(2) INFORMATION FOR SEQ ID NO:1:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
      CCCCAGCTGC AGCCATGAAG 20
(2) INFORMATION FOR SEQ ID NO:2:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
      CCCCAGGGAT ATCCCTGCAG CCATGAAG 28
(2) INFORMATION FOR SEQ ID NO:3:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
      GCTAGGTACC ATGTTTTCCA TGAGGATOGT 30
(2) INFORMATION FOR SEQ ID NO:4:
   (i) SEQUENCE CHARACTERISTICS:
      (A) LENGTHs 30 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (xi) SEQUENCE DESCRIPTION: SEQ ID NO:4:
      GATTTAGAGC GGGACAGATC CCATGGTGAC 30
(2) INFORMATION FOR SEQ ID NO:5:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO.5.
      GCTAGGTACC ATGAAAAGAA TGGTTTCGTG 30
(2) INFORMATION FOR SEQ ID NO:6:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (xi) SEQUENCE DESCRIPTION: SEQ ID NO:6:
      GGAAGAAGGG TGTCGTTATC CCATGGTGAC 30
(2) INFORMATION FOR SEQ ID NO:7:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
      GCTAGGTACC ATGAGTTGGT CCTTGCACCC 30
(2) INFORMATION FOR SEQ ID NO:8:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iv) ANTI-SENSE: YES
   (xi) SEQUENCE DESCRIPTION: SEQ ID NO:8:
      TTGTCCGACC TCTGCAAATT CCATGGTGAC 30
(2) INFORMATION FOR SEQ ID NO:9:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:9:
      CTGTGTGGCC AAGAAGGAAG TGTTG 25
(2) INFORMATION FOR SEQ ID NO:10:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION : SEQ ID NO:10:
      CCCGATAGCC ACCTCCTCTG ATG 23
(2) INFORMATION FOR SEQ ID NO:11:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:11:
      CCCGATAGCC ACCTCCTCTG ATG 23
(2) INFORMATION FOR SEQ ID NO:12:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:12:
      CCTGGACTTC AAAGTAGCAG CGTC 24

## Description

### BACKGROUND OF THE INVENTION

### Field of the Invention

This application relates in general to the use of non-human transgenic animals to produce therapeutically useful amounts of clinically important proteins. More particularly, this application relates to the production in transgenic animals of clinically useful quantities of the blood clotting protein, fibrinogen ("FIB").

### Description of the Background Art

The ultimate event in the blood clotting cascade is the thrombin-catalyzed conversion of FIB (Mr=340,000) to fibrin (Mr=329,000), the latter forming the fibrin clot. FIB deficiency is generally transmitted as an autosomal recessive trait and may manifest as a complete or partial absence of FIB from the blood plasma. Clinically, the disease resembles moderate or mild hemophilia. Congenital fibrinogen abnormality may be due to the hereditary synthesis of structurally or functionally abnormal molecules, as in Vlissingen, Ijmuiden and Nijmegen fibrinogens. An acquired deficiency of this protein may occur as the result of impaired hepatic synthesis of the protein as, for example, in hepatitis or hepatic necrosis, or to accelerated destruction of the protein caused, for example, by increased blood proteolytic activity.

Control of bleeding in such patients is currently achieved by transfusion of FIB contained in freshly-frozen human plasma or in concentrates of the protein isolated from donor blood. While these replacement therapies are generally effective, they place patients at risk for virus-transmissible diseases such as hepatitis or AIDS. Although this risk has been greatly reduced by inactivating such viruses with heat or orgaric solvents, such preparations have greatly increased the cost of treatment, and are not risk free. There is thus a critical need for a source of this protein alternate to human plasma.

An important advance in obtaining an alternate clinical source of FIB has been the cloning of cDNAs encoding the three different fibrinogen chains, and the publication of cDNA sequences. Rixon et al., Biochemistry 22: 3237 (1983); Chung *et al., ibid* 3244; Chung *et al*., *ibid* 3250. The structure of the FIB molecule is exceedingly complex. Each molecule of FIB consists of two sets of three different polypeptide chains, designated Aα, Bβ and G**γ**, with molecular masses of 66 kDa, 52 kDa and 46.5 kDa, respectively. The two half-molecules containing each set of chains are linked together by three disulfide bonds. In addition, a complex set of intra- and interchain disulfide bonds (there are a total of 29 disulfide bonds with no free sulfhydryl groups) are involved in maintaining proper functional structure. Further, FIB is a glycoprotein with highly specific glycosylations. The molecule contains four carbohydrate chains, one each on the B, β, G and γ chains; the α and A chains contain no carbohydrate. About 11 kDa of the total molecular mass of FIB (340 kDa) is attributable to this carbohydrate, added to the molecule post-translationally. In addition, isoforms of glycoproteins are known corresponding to differences in sialic acids on the carbohydrate chains. Proper carbohydrate modification is required for functional activity of FIB.

These highly complex characteristics of the functional FIB molecule has made unpredictable and difficult the expression, assembly and secretion of fully formed and functional recombinant molecules. Although the cDNA encoding the human FIB Aα chain has been expressed in bacteria (Lord, DNA 4:33 (1985)) this is of limited usefulness as the other fibrinogen chains that bear carbohydrates cannot be produced in prokaryotes.

Individual FIB chains have been expressed in COS1 (transformed monkey kidney fibroblast) cells. Danishevsky et al., Biochim. Biophys. Acta 1048:202 (1990). In addition, transfecting COS1 cells with a combination of cDNAs encoding the individual human fibrinogen subunit chains is reported to produce the holoprotein, but the amounts produced were small, and substantially less than the production achieved in the transgenic animal systems to be described below. Roy et al., J. Biol. Chem., 266:4758 (1991). The secretion of partially assembled or wholly unassembled and separate human FIB ("hFIB") or recombinant human FIB ("rhFIB") chains has not been reported for native or genetically engineered tissues. Chung *et al*. (1983), Danishevsky *et al* (1990). In addition, there are serious drawbacks to the use of mammalian cell tissue culture systems for production of FIB. These include the high costs of growth media, the labor intensive nature of such systems, and limited production capacity.

An important need persists for an efficient and relatively inexpensive means of producing clinically useful amounts of infectious particle-free rhFIB protein. The present invention satisfies this need. It has been surprisingly found that transgenic animals can be genetically engineered to produce and secrete into readily accessible body fluids therapeutically useful quantities of rhFIB. In addition to therapeutic uses involving replacement or addition therapy, the FIB of the invention finds us in a variety of applications, such as a "glue" in surgical procedures, as a delivery system for drugs, such as antibiotics or anti-parasitic agents, to wounds, as a food substitute, and for altering the composition of milk. These transgenic systems are described below.

### SUMMARY OF THE INVENTION

In accordance with the present invention, there is provided a non-human mammalian animal that has stably integrated into its genome heterologous, *i.e.*, exogenously derived, polynucleotides that encode the Aα, Bβ and Gγ polypeptide chains of human FIB, and that direct by means of regulatory and signal sequences the expression of biologically active rhFIB in mammary gland cells, such that newly synthesized fibrinogen is secreted into fluid compartments, particularly milk, of the animal. By integrating fewer than all of the three heterologous polynucleotides, individual chains of fibrinogen can be produced and may be secreted. By integrating heterologous polynucleotides that have been modified prior to administration to the host animal, modified FIB and products thereof can be produced.

It therefore is an object of the present invention to provide transgenic animals capable of producing rhFIB, polypeptide subunit chains of FIB, and FIB-derived proteins and protein products.

It is also an object of the invention to provide a means of producing rhFIB, polypeptide subunits thereof, and FIB-derived proteins and protein products in transgenic animals.

In a preferred embodiment of this aspect of the invention, lactating transgenic animals produce the rhFIB, FIB subunits and FIB-derived proteins in their mammary glands and secrete these products in their milk.

In another preferred embodiment, transgenic animals secrete the produced rhFIB, FIB subunits, and FIB-derived proteins in their blood and/or urine.

### BRIEF DESCRIPTION OF THE FIGURES

Figure 1 is a sketch of the murine WAP gene.
Figure 2 is a sketch of the plasmid pUCWAP4 cassette vector containing - 2.6 kbp of WAP 5' promotor region, -1.3 kbp of WAP 3' UTR and flanking 3' region.
Figure 3 is a sketch of the plasmid pUCWAP5 cassette vector containing the WAP fragment of pUCWAP4 with added Not I linkers.
Figure 4 is a sketch of the pUCWAP5 plasmid incorporating a polynucleotide encoding the FIB Aa 1 chain.
Figure 5 is a sketch of the pUCWAP5 plasmid incorporating a polynucleotide encoding the FIB Bβ 1 chain.
Figure 6 is a sketch of the pUCWAP5 plasmid incorporating a polynucleotide encoding the FIB Gγ 1 chain.
Figure 7 shows the family tree of transgenic mice that have incorporated into their genome DNAs encoding FIB genes.

### DETAILED DESCRIPTION OF THE INVENTION

Applicant has discovered a means of producing non-human transgenic mammalian animals that are genetically engineered to secrete into readily accessible body fluids such as milk, blood and urine recombinant human FIB ("rhFIB"), individual subunit chain polypeptides, and modified FIB subunit chains in amounts and in forms that are suitable for treating humans with genetic or acquired deficiencies of the normal protein.

The term "animal" here denotes all mammalian animals except humans. It also includes an individual animal in all stages of development, including embryonic and fetal stages. A "transgenic" animal is any animal containing cells that bear genetic information received, directly or indirectly, by deliberate genetic manipulation at the subcellular level, such as by microinjection or infection with recombinant virus.

"Transgenic" in the present context does not encompass classical crossbreeding or *in vitro* fertilization, but rather denotes animals in which one or more cells receive a recombinant DNA molecule. Although it is highly preferred that this molecule be integrated within the animal's chromosomes, the invention also contemplates the use of extrachromosomally replicating DNA sequences, such as might be engineered into yeast artificial chromosomes.

The term "germ cell line transgenic animal" refers to a transgenic animal in which the genetic information has been taken up and incorporated into a germ line cell, therefore conferring the ability to transfer the information to offspring. If such offspring, in fact, possess some or all of that information, then they, too, are transgenic animals.

The information to be introduced into the animal is preferably foreign to the species of animal to which the recipient belongs (i.e., "heterologous"), but the information may also be foreign only to the particular individual recipient, or genetic information already possessed by the recipient. In the last case, the introduced gene may be differently expressed than is the native gene.

The transgenic animals of this invention may be any other than human, that produce milk, blood serum, and urine. Farm animals (pigs, goats, sheep, cows, horses, rabbits and the like), rodents (such as mice), and domestic pets (for example, cats and dogs) are included in the scope of this invention.

It is highly preferred that the transgenic animals of the present invention be produced by introducing into single cell embryos appropriate polynucleotide that encode human FIB, or subunit chain polypeptides or modified products thereof, in a manner such that these polynucleotides are stably integrated into the DNA of germ line cells of the mature animal and inherited in normal mendelian fashion.

Advances in technologies for embryo micromanipulation now permit introduction of heterologous DNA into fertilized non-human mammalian ova. For instance, totipotent or pluripotent stem cells can be transformed by microinjection, calcium phosphate mediated precipitation, liposome fusion, retroviral infection or other means, the transformed cells are then introduced into the embryo, and the embryo then develops into a transgenic animal. In a preferred method, developing embryos are infected with a retrovirus containing the desired DNA, and transgenic animals produced from the infected embryo. In a most preferred method, however, the appropriate DNAs are coinjected into the pronucleus or cytoplasm of embryos, preferably at the single cell stage, and the embryos allowed to develop into mature transgenic animals. Those techniques as well known. For instance, reviews of standard laboratory procedures for microinjection of heterologous DNAs into mammalian fertilized ova include: Hogan et al., Manipulating the Mouse Embryo, Cold Spring Harbor Press, 1986; Krimpenfort et al., Bio/Technology 9: (1991); Palmiter et al., cell, 41:343 (1985); Kraemer et al., Genetic Manipulation of the Early Mammalian Embryo, Cold Spring Harbor Laboratory Press, 1985; Hammer et al., Nature, 315:680 (1985); Wagner *et al.*, U.S. 5,175,385; Krimpenfort *et al.,* U.S. 5,175,384, all of which are incorporated by reference. The human FIB or FIB subunit genes can be obtained by isolating them from an appropriate genomic source (i.e., human liver which is the natural organ for production of this protein) by alternate methods which include preparation of cDNAs from isolated mRNA templates, direct synthesis, or some combination thereof. The cDNAs encoding individual FIB chains can be fused, in proper reading frame, with appropriate regulatory signals as described in detail below, to produce a genetic construct that is then amplified, for example, by preparation in a bacterial vector, according to conventional methods (see, Sambrook et al, Molecular Cloning: A Laboratory Manual, Cold Spring Harbor Press, 1989 which is incorporated by reference). The amplified construct is thereafter excised from the vector and purified for use in producing transgenic animals. Purification can be accomplished by means of one or more cycles of anionic HPLC; alternate techniques include ultracentrifugation through a sucrose or NaCl gradient, gel electrolution followed by agarose treatment and ethanol precipitation, or low pressure chromatography. Purification by several cycles of HPLC allows for remarkably high transformation frequencies, on the order of 20% or more in both mice and pigs.

Although the present invention preferably entails the use of DNA constructs that produce the desired or native human FIB *per se*, the desired protein may also be produced as a fusion protein containing another protein. For example, the desired recombinant protein of this invention may be produced as part of a larger recombinant protein in order to stabilize the desired protein or to make its purification from milk faster and easier. The fusion partners are then separated chemically or enzymatically, and the desired FIB isolated. Production of a fusion product between FIB and β-lactoglobulin (BLG) is described in detail below.

The rhFIB may also be produced having the identical sequence as the native molecule, or it may be produced as a fragment or derivative. A variety of modified rhFIB or subunits thereof can be produced by altering a cloned DNA using the well-known techniques of *in vitro* mutagenesis such as those set out in the references above.

Production of transgenic animals containing the gene for human FIB involves the use of cDNA or genomic DNA that encodes the α, β, and **γ** chains of hFIB, Rixon *et al*., 1983, above; Chung et *al*., 1983, above. The full length base sequence of each chain is provided in these references, which are incorporated by reference.

DNA constructs useful in the present invention provide a double stranded DNA sequence encoding FIB operably linked to all of the *cis*-acting signals necessary for mammary gland-specific expression of this protein, post-translational glycosylation of two of the three sets of chains of FIB (Bβ and Gγ), secretion of FIB into milk or other body fluids, and expression of full biological activity. As set out above, polynucleotides encoding FIB or FIB subunit chains suitable for use in the invention can be isolated by standard techniques starting from the known polynucleotide sequences.

Modified FIB DNA sequences also can be employed in this invention. Useful modifications within this context include, but are not limited to, those that alter post-translational modifications, size or active site of FIB, or that fuse this protein or portions thereof to another protein. Such modifications can be introduced into the protein by techniques well known in this art, such as by synthesizing modified genes by ligation of overlapping oligonucleotide or introducing mutations into the cloned genes by, for example, oligonucleotide-mediated mutagenesis.

The cis-acting regulatory regions useful in the invention include the promoter that drives expression of the FIB or FIB subunit chain genes. Highly preferred are promoters that are specifically active in mammary gland cells and that involve milk proteins. Among such promoters, highly preferred are the whey acidic protein (WAP), short and long α, β and kappa casein, α-lactalbumin and β-lactoglobulin ("BLG") promoters. Promoters may be selected on the basis of the protein compositions of various milks.

For example, the WAP and BLG promoters are particularly useful with transgenic rodents, pigs and sheep. The rodent WAP short and long promoters have been used to express the rat WAP gene, the human tPA gene and the CD4 gene, while the sheep BLG promoter has been used to express the sheep BLG gene, the human alpha-1-antitrypsin gene and the human Factor IX gene. For reviews, see Paleyanda et al., Recombinant Technology in Hemostasis and Thrombosis, eds. Hoyer et al., Plenum Press, NY 1991, page 197; Clark et al., TIBTECH, 5:20 (1987). which are incorporated by reference. Preferred among the promoters for carrying out the present invention are the rodent casein and WAP promoters, and the casein, α-lactalbumin and BLG promoters from porcine, bovine, equine and ovine (pigs, sheep, goats, cows, horses), rabbits, rodents and domestic pets (dogs and cats). The genes for these promoters have been isolated and their characterizations published; for reviews see Clark *et al*. (1987), above, and Henninghausen, Protein Expression and Purification, 1:3 (1990), which are incorporated herein by reference.

The promoter may be isolated by carrying out conventional restriction endonuclease and subcloning steps. A mouse WAP promoter, isolated as a 2.6 kb EcoR1-Kpn1 fragment immediately 5' to the WAP signal sequence, is preferred, although the "long" WAP promoter (the 5' 4.2 kb Sau 3A-Kpn1 promoter of the mouse WAP gene, or a fragment thereof) is also suitable for carrying out this invention.

Important to the present invention are regulatory sequences that direct secretion of proteins into milk and/or other body fluids of the transgenic animal. In this regard, both homologous and heterologous regulatory sequences are useful in the invention. Generally, regulatory sequences known to direct the secretion of milk proteins, such as either signal peptides from milk or the nascent target polypeptide, can be used, although signal sequences can also be used in accordance with this invention that direct the secretion of expressed proteins into other body fluids, particularly blood and urine, such as signal sequences for coagulation proteins such as protein C and Factor VIII. Most preferred for the transgenic mouse is the regulatory sequences for the WAP protein.

Among the useful sequences that regulate transcription, in addition to the promoters discussed above, are enhancers, splice signals, transcription termination signals, and polyadenylation sites. Particularly useful in this regard are those that increase the efficiency of the transcription of the genes for fibrinogen in the mammary gland cells of the transgenic animals listed above. Preferred are transcription regulatory sequences for proteins highly expressed in the mammary gland cells (see above).

Preferably, the expression system or construct of this invention also includes a 3' untranslated region downstream of the DNA sequence encoding the desired recombinant protein, or the milk protein gene used for regulation. This region apparently stabilizes the RNA transcript of the expression system and thus increases the yield of the desired protein. Among the 3' untranslated regions useful in this regard are sequences that provide a poly A signal. Such sequences may be derived, e.g., from the SV 40 small t antigen, the casein 3' untranslated region, or other 3' untranslated sequences well known in this art. Preferably, the 3' untranslated region is derived from a milk-specific protein. The stabilizing effect of this region's poly A transcript is important in stabilizing the mRNA of the expression sequence. Also important in increasing the efficiency of expression of FIB are ribosome binding sites. Likewise, sequences that regulate the post-translational modification of FIB subunits are useful in the invention.

Thus, in accordance with this invention, a double-stranded chimeric DNA including genes encoding the FIB subunit chains operably linked to cis-acting regulatory sequences that promote efficient expression of the former in milk and/or other body fluids are introduced into an embryo where they are integrated into the embryonic genome and become part of the inheritable genetic endowment of all of the animals cells, including the germ line cells, of the adult that matures from the embryo. The FIB thus expressed and secreted into milk is immunologically reactive, as measured by an ELISA assay as described below.

Where the synthesis of a FIB subunit chain may be limiting in the production of the holoprotein, expression of this chain an be increased by placing the gene in a different genomic locus. This other locus can contain a DNA sequence under the same or a different regulatory sequence than the other two FIB sequences.

In a particularly preferred embodiment the transgenes of the invention generally consist of milk protein upstream and downstream and flanking sequences in combination, separately, with each of the three translated portions of the cDNA sequences representing the three FIB subunit chains. A native 5'-WAP regulatory sequence ending in an accessible restriction site immediately before/at the ATG codon may be ligated to each of the respective restriction sites which occur at the ATG of translatable sequences with no linker sequences derived from the chains of human FIB. A restriction site may be made through site mutagenesis of three bases located in each 3'-untranslatable sequences of the three FIB cDNAs. This preserves the stop sequence while adding a restriction site immediately after each of the translatable sequences of the FIB cDNAs. Each of the combined 5'-regulatory and FIB translatable sequences ending in a particular restriction site may then be ligated to a corresponding restriction site which occurs at the beginning of the 3'-untranslated region of WAP and adjoining WAP 3'-flanking region. This construction motif enables native 5'-regulatory and 3'-UTR of the milk protein genes to be immediately juxtaposed without intervening sequences (but see above relating to the insertion of the Bβ gene at a different locus). Particular restriction sites at the ends of all constructs may be selected in order to facilitate concatenation of Aα, Bβ and Gγ constructs into a single domain within the animal genome.

Although the above general descriptions of the DNA constructs of the invention have been given in terms of the WAP promoter, it is emphasized that other suitable promoters (see above for discussion) may be ligated to the fibrinogen encoding polynucleotides in a similar manner. By way of illustration, the following discussion describes the use of the bovine lactoglobulin ("BLG") promoter to increase the efficiency of expression of FIB and FIB-BLG fusion proteins in mammary glands.

Using techniques described above, fibrinogen encoding cDNA may be inserted into an ovine BLG gene. For instance, in order to produce such a construction, the 11.2 Kbp ovine BLG gene may be modified to possess a unique EcoRV site upstream of the initiator ATG codon in the vector pUCXSRV. The sequence around this region is changed as follows:

PvuI MetLys
Seq. ID No. 1 Natural CCCCAGCTGCAGCCATGAAG

EcoRV MetLys
Seq. ID No. 2 pUCXSRV CCCCAGGGATATCCCTGCAGCCATGAAG

This allows the cloning of blunt end fragments upstream of the BLG gene. The 1.5 kbp fragment from a plasmid (*e.g*., pWAP FIB) containing a cDNA encoding human fibrinogen is isolated, blunt ends are generated with T4 DNA polymer, and the product is ligated to EcoRV-cleaved pUCXSRV. Following transformation of *E. coli* with this plasmid, clones that are produced can be characterized by restriction analysis of plasmid DNA prepared by a mini-prep method and by determination of the nucleotide sequence around the 5' and 3' cloning junctions for the DNA. Clones having the desired structure can be used to produce transgenic rodents, pigs, sheep, cows, horses and other farm animals and domestic pets (cats and dogs) that secrete a FIB-BLG fusion product into their biological fluids as described below.

A human fibrinogen genomic sequence also may be fused to the ovine BLG promoter illustrated in the following discussion. DNA sequences encoding ovine BLG in plasmid pUCXSRV are deleted to generate a vector containing only ovine BLG promoter sequences (pUCSV) . As with pUCSRV, blunt ended fragments may be fused to this promoter region by ligation to a unique EcoRV site. The sequences 5' to this site are identical in both plasmids.

Genomic FIB sequences of greater than about 15 kbp can be introduced into transgenic animals, despite their length, through the use of cosmids with overlapping FIB sequences, whereupon the necessary assembly of an entire genomic polynucleotide encoding hFIB is achieved by homologous recombination in vivo after microinjection into an embryo cell. In constructs useful in the foregoing example, a plasmid in which the FIB genomic sequences are fused to ovine BLG 3' flanking sequences just after the fibrinogen translation termination codon to ensure proper transcription, termination and polyadenylation. The hFIB gene fused to ovine BLG 3' flanking sequences is excised from the plasmid, the 3' overhangs repaired using Klenow enzyme, and the product ligated to EcoRV-cleaved pUCSR. Following transformation of *E*. *coli,* the resulting clones are characterized by restriction DNA analysis and by determining the nucleotide sequences around the 5' and 3' cloning junctions. Clones having the desired structure may be introduced into fertilized animal ova for production of transgenic animals.

A variety of vectors based on the BLG gene may be constructed. In constructs based on this approach, the sequences encoding the ovine BLG protein are deleted, but the 5' promoter sequences are retained. Each may possess a different complement of introns from the ovine gene and a unique EcoRV site allowing the cloning of blunt ended fragments between the promoter and 3' flanking region of the gene. However, each contains the BLG promoter, the EcoRV site and the BLG 3'-flanking sequence. For each recombinant, the 1.5 kbp KpnI fragment from pWAP FIB is isolated, blunt ends generated as above, and the product ligated to EcoRV-cleaved vector sequences. Those constructs with the proper structures (determined as described above) may be used to express fibrinogen in the biological fluids of transgenic animals.

We have observed that doubly-transgenic mice produced having native BLG genomic sequences which are injected as separate constructs to be concatenated in vivo as additional flanking sequences to the BLG target cDNA construct (such as, BLG promoter-Intron I-EcoRV-Intron VI-BLG 3' flank plus BLG) give higher expression more frequently than that observed with the use of constructs of the BLG promoter-FIB cDNA-BLG gene or BLG promoter-FIB genomic (± BLG 3' end). Intact or native BLG genomic sequences that are preligated to the BLG-cDNA target may give the same advantage. This same principle can be extended to WAP genomic sequences.

Obtaining milk from a transgenic animal according to the present invention is accomplished by conventional means. See, e.g., McBurney et al., J. Lab. Clin. Med. 64:485 (1964); Velander et al., Proc Natl. Acad. Sci., USA 89:12003 (1992), which are incorporated by reference. Fibrinogen or subunit chains or protein products thereof can be isolated and purified from milk or urine by conventional means without deleteriously affecting activity. A highly preferred method consists of a combination of anion exchange and immunochromatographies, cryoprecipitations, zinc ion-induced precipitation of either whole milk or milk whey (defatted milk) proteins. For these techniques, see Bringe *et al*., *J*. *Dairy Res.* 56:543 (1989) which is incorporated herein by reference.

Milk is known to contain a number of proteases that have the potential to degrade foreign proteins. These include in the main an alkaline protease with tryptic and chymotryptic activities, a serine protease, a chymotrypsin-like enzyme, an aminopeptidase and an acid protease. Clark *et al.* (1987) above. It may be desirable therefore to protect newly secreted FIB or subunit chains thereof against proteolytic degradation. Such precautions include rapid processing of the milk after collection and addition to the milk of well known inhibitors of proteolysis, such as are listed in Sigma Chemical Co. Catalog, 1993 edition, page 850, incorporated herein by reference.

We have used two sandwich ELISA formats to detect and quantify FIB in milk whey. One technique is a polyclonal antibody capture/FIB/monoclonal antibody detection system ("P/M") and the other which uses polyclonal antibodies both for capture and for detection ("P/P"). Both methods are described below. The P/M system benefits from greater specificity for hFIB as it recognizes only one epitope of about 6 to 20 amino acids in length specific to hFIB. However, because the P/M system recognizes only a single epitope, detection sensitivity is reduced. In contrast, the P/P system will provide less specificity as it recognizes many epitopes, but at the same time will provide greater detection sensitivity. As these multiple epitopes include epitopes conserved between mouse and human FIB, the background "FIB" signal is increased when measuring rhFIB in mouse milk by the P/P system. Detailed sandwich ELISA procedures are provided in EXAMPLE 7 below.

The present invention is further described by reference to the following illustrative examples.

### EXAMPLE 1

### WAP-Fibrinogen cDNA Constructs For Expressing Fibrinogen In Transgenic Animals

### Construction of cassette vectors

Fibrinogen subunit chain DNAs, tissue-specific promoters, and secretion signals were obtained from sources described above. Fibrinogen subunit chain cDNAs were cloned into a modified pUC 18 vector, and grown up in *E*. *coli* JM109.

A pUC18 vector (GIBCO-BRL, Gaithersburg, MD) was digested with HindIII + EcoRI restriction endonucleases, blunted with T4 DNA polymerase in the presence of 100 mM dNTPs, and a Not I linker was ligated into the former HindIII-EcoRI multiple cloning site. This modified pUC fragment was additionally digested with Not I+ enzyme to remove extra (multiple copies) NotI linker sequences arising from ligation, and then religated and grown up in *E. coli* JM 109. This procedure modified the pUC18 vector by removing the entire multiple cloning region of pUC18 (including the Kpn I site) and replacing it with a Not I restriction site. The new vector was designated pUCNot1+.

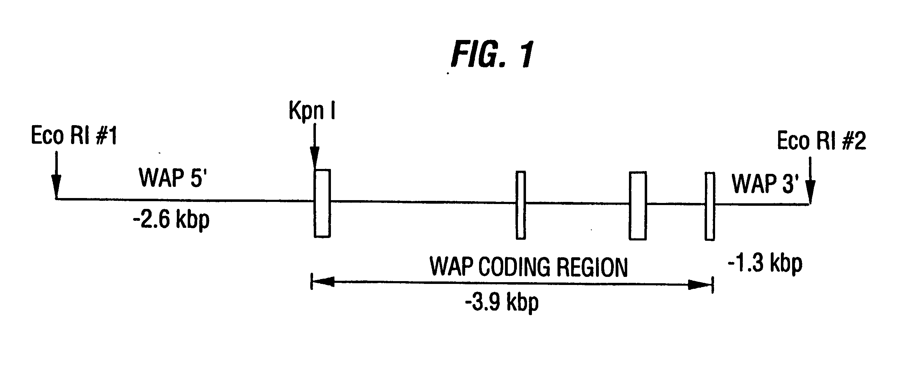

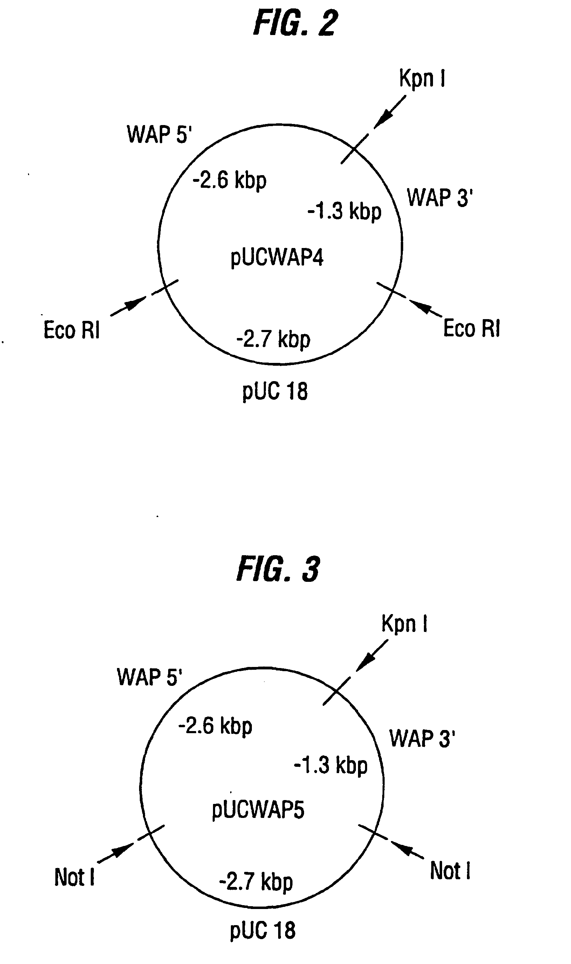

A pUC vector containing -2.6 kbp of WAP 5' promoter region, -1.3 kbp of WAP 3' UTR and flanking 3', but no WAP coding or intronic regions was constructed (designated cassette vector pUCWAP4, Figure 2; see WAP gene, Figure 1, and cassette vector pUCWAP5, Figure 3). The entire murine WAP gene including 2.6 kb of 5' untranslated sequence and 3' untranslated region may be obtained from Dr. I. Hennighausen, National Institutes of Health, Bethesda, Maryland. See, Campbell et al., Nucleic Acids Res., 12:8685 (1984). The WAP fragment contained within the WAP4 vector contains a -2.6 kbp WAP promoter 5' region beginning at EcoRI#1 and ending at the translational start site of WAP which is immediately downstream of the unique Kpn I endonuclease restriction site (see Figure 1). To this Kpn I site was ligated a fragment of -1.3 kbp of WAP UTR and flanking 3' sequence. This WAP 3' DNA included the region from immediately downstream of the WAP stop codon down to the EcoRI#2 site. The WAP fragment contained in WAP4 was excised from the pUC vector using EcoRI, and then blunted, and NotI linkers were added, further trimmed by NotI digestion, and ligated into the pUCNotI+ plasmid which had been linearized with NotI restriction endonuclease. The resulting plasmid was designated pUCWAP5 (see Figure 3).

### Amplification by PCR of fibrinogen subunit chain cDNAs

Each of the cDNAs for Aα, Bβ, and Gγ chains for human fibrinogen were individually modified and amplified by polymerase chain reaction (PCR) to create KpnI endonuclease restriction sites on their 5' and 3' ends. The 5' KpnI site was engineered by PCR using the primers [containing the 6 base sequence (GGTACC shown underlined in Table 1)] that immediately flanks the ATG start codon in the cDNAs of Aα, Bβ, and Gγ chains for hFIB. After amplification, the ends of the extension using PCR products were blunted by T4-polymerase (in the presence of deoxynucleosidetriphosphates to inhibit processive exonuclease activity. In a similar fashion, a KpnI site was engineered by site modification into the 6 base sequence GGTACC shown underlined in Table 1 immediately flanking the stop sequence in the 3' UTR of each cDNA for Aα, Bβ, and Gγ chains for hFIB. The complement of the stop sequences is shown in bold in the 3' primers in Table 1.

**Table 1**

| **Oligonucleotides for Amplifying FIB Subunit Chain cDNA** |
|---|
| Seq. ID No. 3 |
| Aα 5' GCTAGGTACC**ATG**TTTTCCATGAGGATCGT |
| Seq. ID No. 4 |
| Aα 3' CAGTGGTACC**CTA**GACAGGGCGAGATTTAG |
| Seq. ID No. 5 |
| Bβ 5' GCTAGGTACC**ATG**AAAAGAATGGTTTCGTG |
| Seq. ID No. 6 |
| Bβ 3' CAGTGGTACC**CTA**TTGCTGTGGGAAGAAGG |
| Seq. ID No. 7 |
| Gγ 5' GCTAGGTACC**ATG**AGTTGGTCCTTGCACCC |
| Seq. ID No. 8 |
| Gγ 3' CAGTGGTACC**TTA**AACGTCTCCAGCCTGTT |

| |
|---|
| GGTACC = KpnI site ATG = start codon CTA and TTA = stop codon |

### Construction of pUC plasmids containing fibrinogen subunit chain cDNA

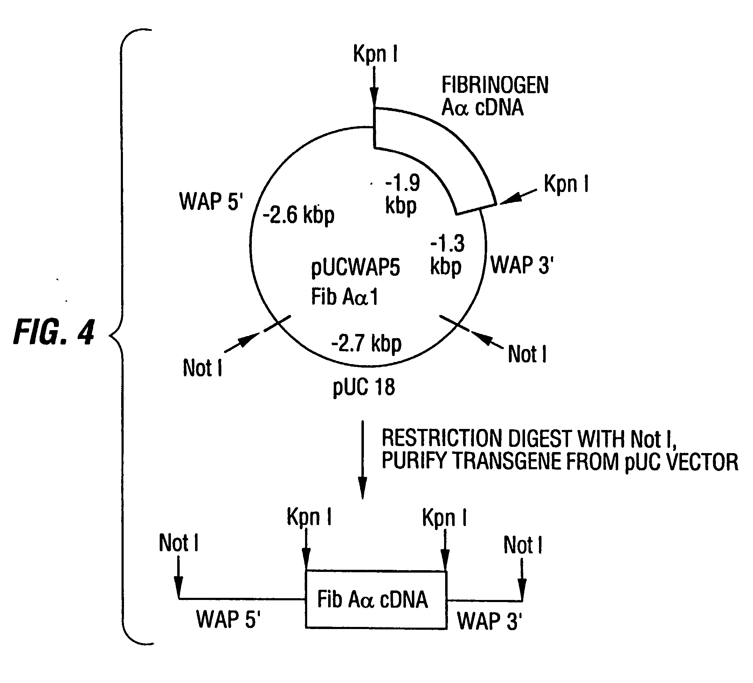

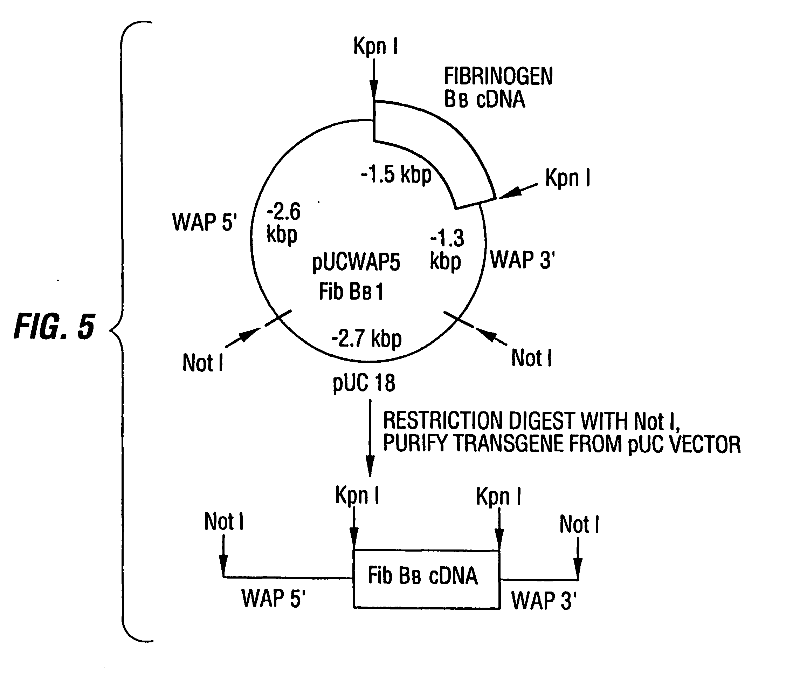

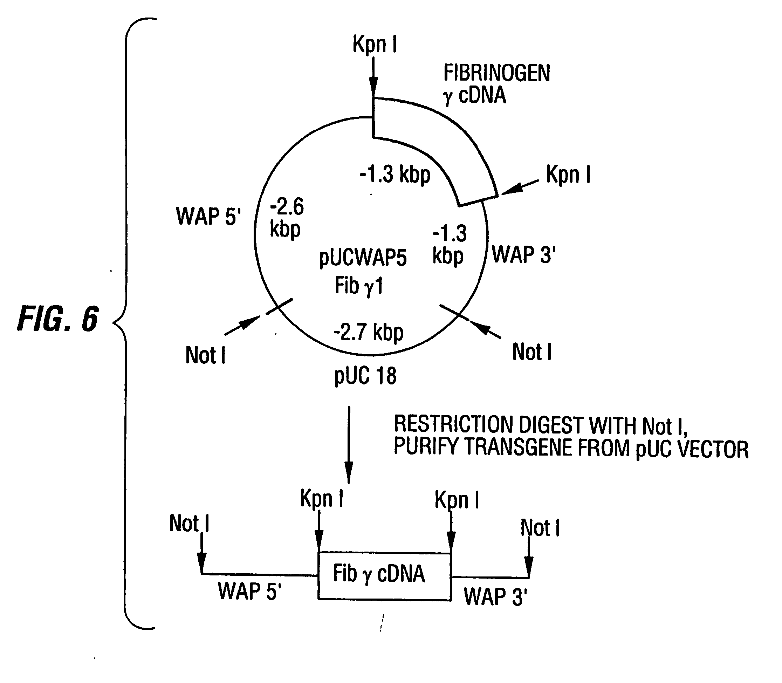

The blunted PCR products of the cDNAs for the Bβ and Gγ chains of human fibrinogen were digested with KpnI restriction endonuclease. In the case of the Aα chain, the PCR product was blunt-end cloned into pUCNotI+ (which had been digested with NotI and blunted with T4 Polymerase) prior to partial KpnI digestion. This intermediate cloning and partial digestion step was necessary to generate intact coding fragment due to the presence of an internal KpnI site within the Aa chain cDNA. The intact Aa chain cDNA fragment was selected by gel electrophoresis, and cloned into the KpnI site at the junction between the WAP 5' promoter and WAP 3' UTR/flanking sequences within the pUCWAP5 plasmid. The KpnI-digested PCR products from Bβ and Gγ chains for human fibrinogen were each directly cloned into a pUCWAP5 plasmid at the KpnI site. Separate electroporation transformation reactions were done on *E.coli* using each of the three pUCWAPS/fibrinogen cDNA preparations, and colonies were picked and grown up in TB ampicillin broth. Plasmid preparations from these colonies were analyzed by restriction enzyme digestion and gel electrophoresis. The correct size and orientations were selected and one clone for each WAP-fibrinogen cDNA construct was sequenced at the WAP promoter 5':fibrinogen cDNA and fibrinogen cDNA:WAP 3' UTR and flanking junctions. Schematics summarizing the construction of the WAP-Aα (about 5.8 kbp), -Bβ (about 5.4 kbp), and -Gγ (about 5.2 kbp) cDNA plasmid and linearized transgenes for human fibrinogen are given in Figures 4, 5, and 6, respectively.

### EXAMPLE 2

### Preparation of DNAs for Microinjection

The intact and linearized WAP 5' promoter/fibrinogen cDNA/WAP 3' UTR and flanking fragments were excised from each pUCWAP5 plasmids by NotI restriction endonuclease digestion and purified by low melting point agarose electrophoresis. The DNA:agarose band was cut from the gel slab. The agarose band was then treated with agarase to degrade and remove agarose contamination

After digestion, the solution containing the cDNA was brought to 10 mM Mg2+, 20 mM EDTA and 0.1% SDS and then extracted with phenol/chloroform. DNA was precipitated from the aqueous layer with 2.5 volumes of ethanol in the presence of 0.3 M sodium acetate at -20° C overnight. After centrifugation, the pellet was washed with 70% ethanol, dried, and each of the constructs was resuspended and dissolved in Brinster microinjection buffer to a total (α, β, plus γ) concentration of about 1.2 to 5 µg/ml. Brinster et al., Proc Natl. Acad. Sci. USA 82:4438 (1985), incorporated by reference.

### EXAMPLE 3

### Transgenic Mice

Transgenic mice were produced essentially as described by Hogan et al., Manipulating The House Embryo, Cold Spring Harbor Press, 1986, incorporated by reference. The procedures employed are outlined below.

Glass needles for micro-injection were prepared using a micropipet puller and microforge. Injections were performed using a Nikon microscope having Hoffman Modulation Contrast optics, with Narashigi micromanipulators and a pico-injector driven by N₂ (Narashigi).

Fertilized mouse embryos were surgically removed from the oviducts of superovulated female CD-1 mice and placed into M2 medium. Cumulus cells were removed from the embryos with hyaluronidase at 300 µg/ml. The embryos were then rinsed in new M2 medium, and transferred into M16 medium for storage at 37° C prior to injection.

Stock solutions containing about 1.2 µg/ml (having about 0.4 µg/ml each of the linearized constructs containing the Aα, Bβ, and Gγ chains cDNAs for human fibrinogen, or about 60-70 copies/pl of each construct) were prepared and microinjected into the pronuclei of 1 cell mouse embryos. In addition, stock solutions containing about 5 µg/ml total DNA (having about 1.7 µg/ml of linearized constructs containing each of the Aα, Bβ, and Gγ chains cDNAs for human fibrinogen or about 200 copies/pl of each construct) were prepared and microinjected into the pronuclei of 1 cell mouse embryos.

After injecting the DNA solution into the male pronucleus, embryos were implanted into avertin-anesthetized CD-1 recipient females made pseudo-pregnant by mating with vasectomized males. About 25-30 microinjected mouse embryos per recipient were transferred into pseudopregnant females. Embryos were allowed to come to term and the newborn mice were analyzed for the presence of the transgene by PCR using murine WAP and a FIB-specific primer as described below.

Table 2 summarizes the embryo transfer data from an experiment that produced transgenic fibrinogen-producing mice. Separate PCR analysis for detection of each of the cDNAs of fibrinogen was done on DNA purified from biopsied tail tissues by the method shown in Example 4 below. The oligonucleotide primers shown in. Table 3 were used to detect each of the FIB cDNAs. Detection of each chain required a WAP oligonucleotide (no. 1 in Table 3) and one of the other oligonucleotides (Nos. 2, 3 or 4).

### Table 2

### Transfers of Fibrinogen Constructs

Thirty transfers with the FIB constructs were performed. The three subunit chain constructs were coinjected at total DNA concentrations of 1.2 *µ*g/ml and 5 *µ*g/ml or 200 total copies/pl and 800 total copies/pl, respectively. Fourteen transfers were made with 1.2 *µ*g/ml-injected embryos and sixteen with 5 *µ*g/ml-inject embryos. Eighteen Founder ("Fo") animals were produced that carried at least one construct. Eight Fo mice were mated with control mice which resulted in five lines transmitting the transgene. First generation offspring ("F1") from lines 1,23 and 85 have been bred and litters produced.

| | 1.2*µ*g/ml | 5*µ*g/ml |
|---|---|---|
| Transfers: | 14 | 16 |
| Pups alive: | 68 | 69 |
| Embryos injected: | 696 | 1098 |
| Embryos transferred: | 480 | 675 |
| Embryos per transferred: | 34.2 | 42.1 |
| Pups per litter | 6.2 | 5.3 |
| Transfers pregnant: | 11 | 13 |
| Pregnancy rate: | 78% | 81% |
| Mice tested: | 68 | 69 |
| Number transgenic: | 7 | 11 |
| Percent transgenic: | 10% | 15% |
| % Positive for α,β,γ: | 85% | 72% |
| α,β,γ Transgenic females: | 3 | 3 |

**Table 3**

| **Oligonucleotide Primers Used for PCR Detection of Human Fibrinogen Transgenic Mouse** |
|---|
| Seq. ID No. 9. Sense strand. WAP-specific oligonucleotides. |
| 5'-CTGTGTGGCCAAGAAGGAAGTGTTG-3' |
| Seq. ID No. 10. Antisense oligonucleotide. Aα chain detection. |
| 5'-GATGTCTTTCCACAACCCTTGGGC-3' |
| Seq. ID No. 11. Antisense oligonucleotide. Bβ chain detection. |
| 5'-CCCGATAGCCACCTCCTCTGATG-3' |
| Seq. ID No. 12. Antisense oligonucleotide. Gγ chain detection. |
| 5'-CCTGGACTTCAAAGTAGCAGCGTC-3' |

A summary of the types of fibrinogen transgenic mice detected in founder and offspring of founder mice is given in Table 4. A listing of the positive FIB triple construct founders (Fo) and a listing of β-chain positive transgenic mice are also given in Table 4.

**Table 4**

| **Transmission of Fibrinogen Transgenes** | | |
|---|---|---|
| Mouse No. | Transmission Frequency | Genes Transmitted |
| 1 (α,β,γ) | 9/13 (69%) | α,β,γ |
| 23 (α,β,γ) | 6/12 (50%) | α,β,γ (3/6); |
| | | β only 3/6) |
| 109 (α,β,γ) | 5/11 (45%) | α,β,γ |
| 112 (α,β,γ) | 5/8 (62%) | α**,**β**,**γ (4/6) |
| | | α only (1/6) |
| | | α, γ only (1/6) |
| 85 (α,β) | 1/6 (16%) | β only |
| 113 (αβγ) | 0/8 | None |
| 89 (α) | 0/6 | None |

Positive Fibrinogen Triple Construct F1's
1-2,1-5,1-6,1-11,1-12,1-14,1-16,1-17,1-20,23-3,23-4,23-12,109-3,109-4,109-5,109-10,109-11,112-3,112-6,112-7,112-8
β-Chain Positive Mice
23-2,23-7,23-11,85-2

All embryos were injected with the three FIB constructs at a total DNA concentration of 5 *µ*g/ml or about 200-300 copies of each construct per pl.

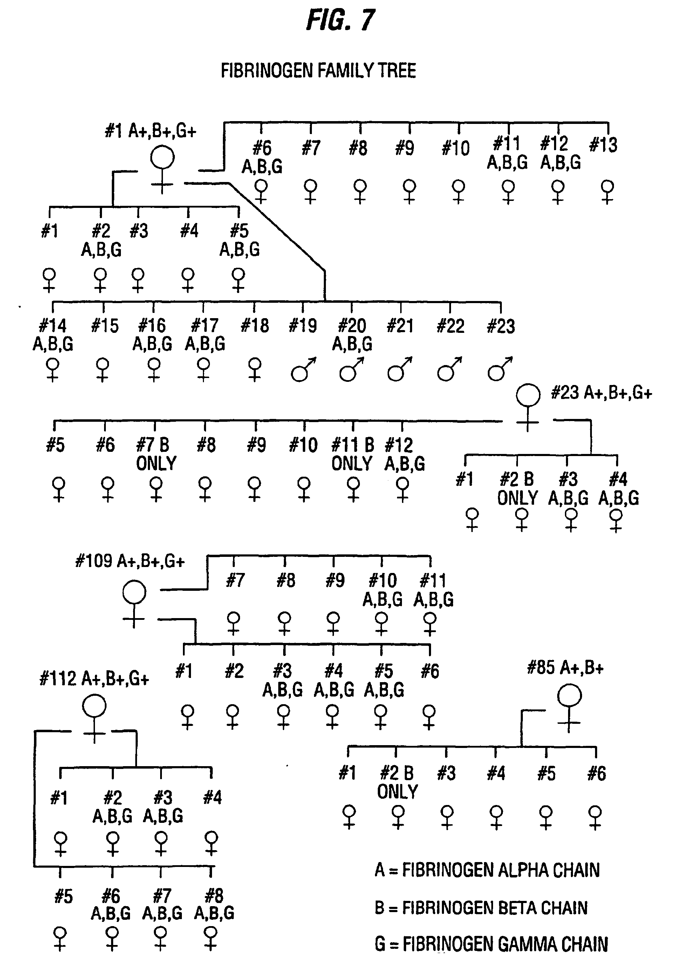

A family tree of transgene passage from F0 to F1 females is shown Figure 7. Males were not retained.

### EXAMPLE 4

### Transgenic Animal DNA

DNA can be prepared from tissue of a transgenic animal of any species by the method exemplified below for mice. Marmur, J. Mol. Biol. 3:208 (1961), incorporated by reference.

A 5 mm piece of mouse tail was removed from young, potentially transgenic mice at weaning (3 weeks) age, and frozen in liquid nitrogen. To the frozen tissue was added 840 *µ*l of Lysing Solution (8 mM EDTA-0.8% SDS-0.8% 2-mercaptoethanol-80 *µ*g/ml Proteinase K-1 M sodium chlorate in 40 mM TRIS buffer pH 8.0 and 120 mM NaCl, and the mixture incubated at 50°C. The mixture was then extracted with 250 *µ*l of phenol/chloroform/isoamyl alcohol (25:24:1) for 10-15 seconds, then centrifuged for 10 minutes. The supernatant fluid (about 830 *µ*l) was removed to a fresh tube, and a DNA clot produced by vortexing the solution with 0.6 vols. of isopropanol. The mother liquor was decanted, and the DNA clot rinsed twice with 80% ethanol. The DNA clot was isolated by 5 mins. of centrifugation, aspiration of the supernatant fluid, and air drying of the clot with a stream of air for 10 mins.

The DNA clot was dissolved in 250 µl of TE buffer (10 mM Tris.HCl, pH 7.0-1mM EDTA, and the solution treated with 10 µl of a RNase mixture (1 mg/ml RNAse A and 4,000 units/ml RNAse T1) for 1 h at 37°C. This mixture was shaken with 50 µl of a 24:1 (v/v) solution of chloroform-isoamyl alcohol for 5-10 secs., centrifuged, and the supernatant fluid transferred to a fresh tube.

The recovered supernatant fluid above was mixed sequentially with 25 µl of 3 M sodium acetate and 0.5 ml of 95% ethanol. The supernatant fluid was decanted from the precipitated DNA, and the precipitate washed with 80% ethanol. The purified DNA was isolated by centrifugation, air dried, then dissolved in 150 µl of TE.

Essentially the same technique was used to prepare DNA from pigs, and the same or similar techniques can be used to prepare DNA from other animals.

PCR reactions on DNA samples isolated from transgenic animal tissue samples were carried out conventionally, as described above. Concentrations of DNA isolates were determined by measuring the absorbancy at 260 nm of 1:20 dilutions of the TE solutions described above. A portion of the sample was then diluted into 100 µl of TE to a concentration of 30 ng/µl.

| PCR reaction mixtures (20 µl volume) | | |
|---|---|---|
| Component | Volume (µl) | concentration |
| HOH | 13.25 | - |
| 10X Taq buffer | 2.5 | 1 |
| dNTPs | 2.0 | 0.2 mM |
| MgCl₂ | 1.5 | 1.5 mM |
| Primer 1 | 0.3125 | 0.5 µM |
| Primer 2 | 0.3125 | 0.5 µM |
| Taq polymerase | 0.125 | 25 µunits/µl |
| DNA template | 1.0 | 1.2 ng/µl |

### Procedure

Embryo lysing buffer (5 *µ*l, 0.9% Nonidet P-40 and 0.9% Tween 20 in 20 mM TRIS buffer) was added to each PCR tube along with 1 *µ*l of prepared DNA solution, and the mixture overlaid with 25 *µ*l of mineral oil. The tubes were placed in an automatic temperature cycler (such as that manufactured by N.J. Research) and heated to 98°C for 2 mins.; the temperature was then brought down to 85°C.
PCR reaction mixture (20 *µ*l) was added to each tube and 40 cycles of the following protocol carried out:
Denaturation at 96°C for 20 seconds
Annealing at 55°C for 1 minute
Elongation at 75°C for 30 seconds
PCR reaction products were analyzed by running 20% of the reaction mixture products on agarose gels and identifying fragment sizes by comparison with marker DNA fragments.

Figure 7 shows that a variety of founder animals having A+ and/or B+ and/or G+ constructs can transmit all three or some of the A+, B+, and G+ transgenes. All animals were produced from embryos having coinjections of the same DNA solution containing all of the A+, B+, and G+ constructs. Detection of A+, B+ and G+ DNAs was done by PCR as described above. Founder animals 1, 23, 109 and 112 had all three transgenes. Founder animal 85 had only A+ and B+. These founder animals were outbred with control (nontransgenic) mice. Animals 1 and 109 passed all three transgenes to 5 of 13 and 5 of 11 offspring, respectively, which is typical of Mendelian genomic transmission for a single allele. Founder animals 23 and 112 transmitted some transgene to 6 of 12 and 7 of 8 animals, respectively. Several offspring of animal 23 had only B+, while one offspring of animal 112 had only A+ and another had A+ and G+. Thus, founder animals 23 and 112 are examples of triple transgenic animals having a confirmed separate B+ allele in the case of animal 23 and having a separate A+ and B+ allele in the case of animal 112. Founder animal 85 is also an example of a mouse with a B+ only allele. Thus, crossbreeding of animals having separate alleles and therefore loci for each gene can be done in order to increase expression (the control over a rate limited B+ chain may be overcome by having a B+ allele with a more inducible loci). Furthermore, double and triple heterozygotes of the same gene (either A+ and/or B+ and/or G+) can be achieved to boost production levels (i.e., produce a A+ B+ G+, B+ animal by crossbreeding a B+ animal, and further crossbreeding that **A+8+G+,B+** animal with a different B+ line to obtain A+ B+ G+,B+ B+).

### EXAMPLE 5

### Transgenic Pigs

Embryos were recovered from the oviduct. They were placed into a 1.5 ml microfuge tube containing approximately 0.5 ml embryo transfer media (phosphate buffered saline + 10% fetal calf serum, Gibco BRL). These were then centrifuged for 12 minutes at 16,000 x g RCF (13,450 RPM) in a microcentrifuge (Allied Instruments, model 235C). Embryos were removed from the microfuge tube with a drawn and polished Pasteur pipette and placed into a 35 mm petri dish for examination. If the cytoplasm was still opaque with lipid such that pronuclei were not visible, the embryos were centrifuged again for 15 minutes. Embryos to be microinjected were placed into a microdrop of media (approximately 100 *µ*l) in the center of the lid of a 100 mm petri dish. Silicone oil was used to cover the microdrop and fill the lid to prevent media from evaporating. The petri dish lid containing the embryos was set onto an inverted microscope (Carl Zeiss) equipped with both a heated stage and Hoffman Modulation Contrast optics (200 x final magnification). A finely drawn (Kopf Vertical Pipette Puller, model 720) and polished (Narishige microforge, model MF-35) micropipette was used to stabilize the embryos while about 1 - 2 picoliters of HPLC-purified DNA solution containing approximately 200-500 copies of DNA construct was delivered into the male pronucleus with another finely drawn micropipette. Embryos surviving the microinjection process as judged by morphological observation were loaded into a polypropylene tube (2mm ID) for transfer into the recipient pig. Seven surgical transfers of pig embryos were made. These embryos had been coinjected with the WAP-fibrinogen cDNA constructs used in the above mentioned transgenic fibrinogen mice Only one of these recipient gilts was confirmed pregnant by ultrasound. The (apparent) non-pregnant animals were kept to tentative term as their (potential) litter sizes may have been too small to detect by ultrasound analysis (In the past, we have had 3 litters of 4 piglets or less which were not detected by ultrasound. Some of these litters produced transgenic animals.)

**Table 5**

| **Fibrinogen Porcine Embryo Data** | | | |
|---|---|---|---|
| Expt. 1 | | Expt. 2 | |
| | | | Transfer Totals |
| | | | 10/25/93 ID |
| Number of donors: | 8 | 8 | Transfer#1 |
| Number of | | | 50 embryos #129 |
| ovulations: | 261 | 283 | Transfer#2 |
| | | | 47 embryos #147 |
| Embryos collected: | 250 | 296 | Transfer#3 |
| | | | 44 embryos #108 |
| Number of UFO 's: | 99 | 77 | |
| UFO's that cleaved: | 29 | 6 | |
| Number injected: | 141 | 219 | 10/28 |
| Number transferred: | 141 | 200 | Transfer#1 |
| | | | 50 embryos #114 |
| Transfers: | 3 | 4 | Transfer#2 |
| | | | 50 embryos #139 |
| Embryos/Transfer: | 47 32.6 | 50 35.3 | Transfer#3 |
| | | | 50 embryos #140 |
| Ovulations/Pig: | | | Transfer#4 |
| | | | 50 embryos #131 |
| Embryos/Pig: | 31.2 | 37 | |

All embryos were injected with the three fibrinogen constructs at a total DNA concentration of 5 µg/ml or about 200-300 copies of each construct per pl.

### EXAMPLE 6

### Preparation of Milk and Whey from Transgenic Animals

Lactating mice were milked once on about day 12 of lactation. The mice were first separated from their young for approximately 5 hours. They were then anesthetized with 0.4'ml avertin at 2.5% (im.), and 0.2 ml oxytocin was then administered at 2.5 IU/ml (ip.) to permit release of the milk. A milking device consisting of a vacuum pump (2.5 psi) and syringe with an Eppendorf tip was used to direct milk into an eppendorf tube. As noted above, milk should not be placed on ice because of cryoprecipitation of fibrinogen.

To prepare whey, milk was diluted 1:1 with TRIS buffer (0.03 M Tris pH 7.4; 0.06 M NaCl) and centrifuged in a TLA-100 rotor in a Beckman TL-100 table top ultracentrifuge at 51,000 rpm (89,000 x g) for 30 minutes at room temperature. After centrifugation, the whey (infranatant fluid) was collected with an 18 gauge needle, leaving the casein pellet and upper cream layer in the tube. To remove solids or cream that co-transferred during the initial recovery, the whey obtained from the first centrifugation was subjected to a second spin at 12,000 rpm for 30 mins. in a TMA-4 rotor in a Tomy MTX-150 centrifuge. Following the second spin, the whey was recovered as before.

### EXAMPLE 7

### Determination of Fibrinogen in Milk by a Sandwich ELISA

A sandwich ELISA was used to measure the amount of FIB protein produced by founder and progenic transgenic animals in the diluted skim or defatted milk (here termed "whey") ; the whey value is used to estimate the concentration of FIB in whole milk.

Generally, in the first step of the P/M ELISA system, a fibrinogen or fibrinogen chain analyte-containing sample (for example, whey) is incubated with a mouse anti-human fibrinogen mAb (clonal number 311, American Diagnostics) that is reactive with the alpha chain of fibrinogen to form a mAb-fibrinogen (or chain) first complex. This first complex is then "captured" by incubation with rabbit polyclonal anti-human fibrinogen antibodies immobilized in microliter plate wells. In the detection step, the captured fibrinogen (or alpha chains) is contacted with labeled (horse radish peroxidase) antimouse IgG, and the amount of label quantified by reaction with o-phenylenediamine and measurement of absorbency at 490 nm; this is a measure of the amount of analyte present. Standard curves were obtained by spiking the whey of control (nontransgenic) animals with pure reference human FIB material. Thus, the presence of background amounts of FIB is normalized by the standard curve. This sandwich ELISA gives very low background values (about 0.06 absorbancy units at 490 nm) for mouse whey alone with no human FIB. Note the typical signals given in Table 6 are about 0.25 or higher absorbancy units at this wave length.

In the P/P system, Immulon II 96-well microliter plates were coated with 100 µl/well of 1:500 diluted rabbit anti-human FIB (Celsus cat. no. 60960) in 0.1 M NaHCO₃-0.1 M NaCl, pH 9.6, for 24 h at refrigerator temperatures. Coated wells were washed three times with Wash Buffer (25 mM Tris-HCl-50 mM NaCl-0.5% Tween-20, pH 7.0) and blocked for 20 mins. with Dilution Buffer (1 mg/ml PEG in 25 mM Tris-HC1-50 mM NaCl, pH 7.0) at room temperature. 100 µl of standard human FIB (100 ng/ml to 1.9 ng/ml range) and/or 100 µl of diluted milk sample were added to each well, and the mixtures incubated at 37°C for 45 mins. The wells were washed and blocked as above for 10 mins. at room temperature. 100 *µ*l of 1:1000 diluted horse radish peroxide-conjugated rabbit anti-human FIB were added to each well, and the mixtures incubated at 37°C for 45 mins. After washing the wells as above, 100 *µ*l of o-phenylenediamine solution (1 tablet/5 ml diluent) were added to each well. After waiting about 2-3 mins. for color development, reactions were stopped by the addition to each well of 100 µl of 3 N H₂SO₄. Plates were then read at 490 nm.

Table 6 presents the results of the analyses of rhFIB by the P/M ELISA system described above in the first lactation whey (and in whole milk by calculation) of three founder (Fo) transgenic mice whose genome contained each of the Aα, Bβ, and Gγ chains cDNAs for human fibrinogen. In this experiment, "whey" refers to skim milk diluted 1.7X with EDTA to solubilize the caseins. The averages of four dilutions of each whey sample for the three test animals was about 16.3, 3.86 and 7.55 *µ*g/ml, respectively. The corresponding values for whole milk were 27.6, 6.56 and 12.8. Second lactation production of rhFIB was similar.

In another experiment with the same animals the first lactation whey fibrinogen concentrations averaged about 22 ± 4 (animal 1), 9 ± 2 (animal 22) and 11 ± 3 (animal 23) µg/ml. The second lactation of animal 1 produced 17 ± 3 µg/ml whey.

The P/M ELISA system was used to assay for rhFIB in the third lactation milk of mouse no. 1/Fo and the first lactation of mouse no. 1 female offspring (1-11/F1). As shown in the data of Table 7, mouse no. 1/Fo had about 71 µg/ml, and her offspring (no. 1-11/F1) had about 53 µg/ml total FIB signal. Thus, the amount of rFIB after subtracting background is calculated to be about 50 µg/ml for mouse no. 1/Fo and about 32 µg/ml for offspring 1-11/F1, respectively, over that of endogenous FIB present from serum to milk passover. The slightly lower value (28 µg/ml) of the P/M assay of the previous lactation of mouse no. 1/Fo is not significantly different than the P/P value when lower detection sensitivity of the former method is taken into account. It is noted that collection of mouse milk is inherently traumatic and leads to serum passover of endogenous mFIB.

In summary, mouse no. 1/Fo (founder) produced rFIB in milk at a level of about 30 µg/ml or greater over 3 lactations as detected by either the P/M or P/P ELISA methods, and has passed this trait on into the F1 generation which, in mouse no. 1-11/F1, produced 32 *µ*g/ml rFIB.

**TABLE 7**

| **Inheritability of Transgenic rhBIB Gene** | | |
|---|---|---|
| Mouse No. | µg rhFIB/ml 5X | µg rhFIB/ml milk |
| | diluted whey | |
| 1/Fo | 14.0 ± 2.0 | 71.0 |
| 1-11/F1 | 10.4 ± 0.8 | 53.0 |
| Control whey | 4.0 ± 0.7 | 21.0 |

These productions are substantially greater than the 2 µg/ml produced in the COS1 system described by Roy *et al*., 1991, above.

Western analysis of control mouse milk using polyclonal anti-human fibrinogen antibody showed about 21 µg/ml of background fibrinogen signal (see Table 7). Western analysis that uses the clonal number 311 or Y18 monoclonal antibody has a lower limit of sensitivity of about 1 µl sample of 100 µg/ml fibrinogen (Phastgel system) or 20 µl of 25 µg/ml in a 3" by 4" SDS-PAGE gel.

**TABLE 6**

| | A | B | C | D | E | WHEY | MILK |
|---|---|---|---|---|---|---|---|
| 1 | Animal | Dilutions | AVG ABS 490nn | | FIB *µ*/ml | AVG FIB *µ*/ml | AVG FIB *µ*/ml |
| 2 | #1 DAY 6 | 100 | 0.623 | 0.132 | 13.2 | | |
| 3 | | 200 | 0.523 | 0.088 | 17.6 | 16.3 | 27.6 |
| 4 | | 400 | 0.434 | 0.049 | 19.4 | | |
| 5 | | 800 | 0.334 | 0.018 | 14.7 | | |
| 6 | | | | | | | |
| 7 | #22 | 100 | 0.374 | 0.022 | 2.16 | | |
| 8 | | 200 | 0.335 | 0.018 | 3.69 | 3.86 | 6.56 |
| 9 | | 400 | 0.273 | 0.014 | 5.73 | | |
| 10 | | 800 | 0.251 | 0.013 | 10.3 | | |
| 11 | | | | | | | |
| 12 | #23 | 100 | 0.477 | 0.067 | 6.75 | | |
| 13 | | 200 | 0.384 | 0.026 | 5.22 | 7.55 | 12.8 |
| 14 | | 400 | 0.338 | 0.019 | 7.44 | | |
| 15 | | 800 | 0.261 | 0.013 | 10.8 | | |

## Claims

1. A transgenic non-human mammalian animal having stably integrated in its genome a first heterologous DNA sequence encoding a fibrinogen Aα subunit polypeptide chain, a second heterologous DNA sequence encoding a fibrinogen Bβ subunit polypeptide chain and a third heterologous DNA sequence encoding a fibrinogen γ subunit polypeptide chain, said animal or the female offspring of said animal being capable of expressing biologically active fibrinogen in the mammary gland cells and secreting said fibrinogen into the milk of said animal.

2. A transgenic non-human mammalian animal as claimed in claim 1, wherein said first, second and third DNA sequences are operably linked to regulatory sequences that direct expression of said first, second and third DNA sequences in mammary gland cells, and secretion of biologically active fibrinogen into milk.

3. A transgenic non-human mammalian animal as claimed in claim 2, wherein said first, second and third DNA sequences are operably linked to regulatory and signal sequences that direct expression of said first, second and third DNA sequences in mammary gland cells, and secretion of biologically active fibrinogen into milk.

4. A transgenic non-human mammalian animal having stably integrated in its genome a first heterologous DNA sequence encoding a fibrinogen Aα subunit polypeptide chain, a second heterologous DNA sequence encoding a fibrinogen Bβ subunit polypeptide chain and a third heterologous DNA sequence encoding a fibrinogen γ subunit polypeptide chain, said first, second and third DNA sequences being operably linked to regulatory sequences that direct expression of said first, second and third DNA sequences in mammary gland cells, and secretion of biologically active fibrinogen into milk.

5. A transgenic non-human mammalian animal being the offspring of a transgenic non-human mammalian animal as claimed in any one of claims 1 to 4 and being capable of expressing biologically active fibrinogen in the mammary gland cells and secreting said fibrinogen into the milk of said animal.

6. A transgenic non-human mammalian animal as claimed in any one of claims 1 to 5 wherein said animal is capable of transferring or transmitting said first, second and third DNA sequences to its offspring in the form of transgenes stably integrated into the genome of said offspring.

7. A transgenic non-human mammalian animal as claimed in any one of claims 1 to 6 wherein said animal is a female.

8. A transgenic non-human mammalian animal as claimed in any one of claims 1 to 6, wherein said animal is a male.

9. A transgenic non-human mammalian animal as claimed in any one of claims 1 to 8, wherein female progeny of said animal produce said biologically active fibrinogen in milk.

10. A process for producing a transgenic non-human animal as defined in any one of claims 1 to 9, said process comprising providing a first heterologous DNA sequence encoding a fibrinogen Aα subunit polypeptide chain, a second heterologous DNA sequence encoding a fibrinogen Bβ subunit polypeptide chain and a third heterologous DNA sequence encoding a fibrinogen γ subunit polypeptide, introducing said first, second and third heterologous DNA sequences into the genome of said mammalian animal.

11. A process as claimed in claim 10, wherein said first, second and third DNA sequences are introduced into a fertilised ovum of a non-human mammalian animal, and said ovum is allowed to develop into a mature transgenic animal.

12. A process as claimed in claim 10 or claim 11, further comprising allowing the said mature transgenic animal to produce offspring.

13. A process as claimed in claim 10 or claim 11, wherein said introducing step comprises coinjecting said first, second and third heterologous DNA sequences into the pronucleus of a fertilized ovum.

14. A process for producing a heterologous biologically active fibrinogen, comprising the steps of:
a) providing a transgenic mammalian non-human animal as claimed in any one of claims 1 to 9;
b) collecting milk from said animal; and
c) isolating or purifying said expressed biologically active fibrinogen protein from said milk.

15. A process as claimed in claim 14 wherein said transgenic animal is the offspring provided by the process of claim 11.

16. A process as claimed in claim 14, wherein said transgenic animal is provided by a process comprising:
introducing first, second and third DNA sequences as defined in any one of claims 1 to 4 into a fertilised ovum of a non-human mammalian animal;
allowing said ovum to develop into a mature transgenic animal;
allowing said animal to produce female offspring that express said first, second and third DNA sequences and produce milk containing biologically active fibrinogen.

17. The animal or process of any one of claims 1 to 16, wherein each of said first, second and third DNA sequences is operably linked to cis-acting regulatory sequences necessary for expression in the mammary gland and secretion into milk.

18. The animal or process of any one of claims 1 to 16, wherein each of said first, second and third DNA sequences is operably linked to regulatory sequences necessary for its expression in the mammary gland of said animal.

19. The animal or process of any one of claims 1 to 18, wherein each of said first, second and third DNA sequences is further operably linked to signal sequences that direct secretion of proteins into milk.

20. The animal or process of any one of claims 1 to 19, wherein said regulatory sequence comprises a promoter selected from the group consisting of a whey acid protein promoter, a casein promoter, a β-lactoglobulin promoter, or an α-lactalbumin promoter.

21. The animal or process of any one of claims 2 to 20, wherein said regulatory sequence is a whey acid protein promoter.

22. An animal or process of any one of claims 1 to 21, wherein said fibrinogen is human fibrinogen.

23. An animal or process of any one of claims 1 to 22, wherein said transgenic non-human mammalian animal is selected from the group consisting of a rodent, rabbit, pig, sheep, goat, cow and horse.

24. The animal or process as claimed in claim 23 wherein said transgenic non-human mammalian animal is a cow.

25. The animal or process as claimed in any one of claims 1 to 24 wherein said first, second and third DNA sequences comprise genomic DNA.

26. The animal or process of any one of claims 1 to 24 wherein said first, second and third DNA sequences comprise cDNA.

27. A non-human transgenic mammalian animal produced by the process as claimed in any one of claims 10 to 13 or 17 to 26, wherein said animal produces milk containing biologically active fibrinogen.

28. Milk obtained from the transgenic non-human mammalian animal of any one of claims 1 to 9, containing biologically active fibrinogen.

## Patentansprüche

1. Ein transgenes nicht menschliches Säugetier mit einer ersten heterologen DNA-Sequenz, die in seinem Genom stabil integriert ist und eine Polypeptidkette einer Fibrinogen Aα Untereinheit kodiert, einer zweiten heterologen DNA-Sequenz, die eine Polypeptidkette einer Fibrinogen Bβ Untereinheit kodiert und eine dritte heterologe DNA-Sequenz, die eine Polypeptidkette einer Fibrinogen γ Untereinheit kodiert, wobei das Tier oder der weibliche Nachwuchs dieses Tieres in der Lage ist, biologisch aktives Fibrinogen in den Brustdüsenzellen zu exprimieren und dieses Fibrinogen in die Milch des Tieres auszuscheiden.

2. Ein transgenes nicht menschliches Säugetier nach Anspruch 1, wobei die erste, zweite und dritte DNA-Sequenz durchführbar mit Ausführungssequenzen verbunden sind, die einen Ausdruck der ersten, zweiten und dritten DNA-Sequenz in Brustdrüsenzellen und eine Sekretion von biologisch aktivem Fibrinogen in Milch anweisen.

3. Ein transgenes nicht menschliches Säugetier nach Anspruch 2, wobei die erste, zweite und dritte DNA-Sequenz durchführbar mit Ausführungs- und Signalsequenzen verbunden sind, die einen Ausdruck der ersten, zweiten und dritten DNA-Sequenz in Brustdrüsenzellen und eine Sekretion von biologisch aktivem Fibrinogen in Milch anweisen.

4. Ein transgenes nicht menschliches Säugetier, mit einer fest in seinem Genom integrierten ersten heterologen DNA-Sequenz, die eine Polypeptidkette einer Fibrinogen Aα Untereinheit kodiert, eine zweite heterologe DNA-Sequenz, die eine Polypeptidkette einer Fibrinogen Bβ Untereinheit kodiert und eine dritte heterologe DNA-Sequenz, die eine Polypeptidkette einer Fibrinogen γ Untereinheit kodiert, wobei die erste, zweite und dritte DNA-Sequenz durchführbar mit Ausführungssequenzen verbunden sind, die Ausdruck der ersten, zweiten und dritten DNA-Sequenz in Brustdrüsenzellen und Sekretion von biologisch aktivem Fibrinogen in Milch anweisen.

5. Ein transgenes nicht menschliches Säugetier, das der Abkömmling eines transgenen nicht menschlichen Säugetiers nach einem der Ansprüche 1 bis 4 ist und in der Lage ist, biologisch aktives Fibrinogen in den Brustdrüsenzellen zu exprimieren und das Fibrinogen in die Milch des Tieres abzusondern.

6. Ein transgenes nicht menschliches Säugetier nach einem der Ansprüche 1 bis 5, wobei das Tier in der Lage ist, die erste, zweite und dritte DNA-Sequenz seinem Abkömmling in der Form von Transgenen, die in dem Genom des Abkömmlings fest integriert sind, zu transferieren oder zu übertragen.

7. Ein transgenes nicht menschliches Säugetier nach einem der Ansprüche 1 bis 6, wobei das Tier weiblich ist.

8. Ein transgenes nicht menschliches Säugetier nach einem der Ansprüche 1 bis 6, wobei das Tier männlich ist.

9. Ein transgenes nicht menschliches Säugetier nach einem der Ansprüche 1 bis 8, wobei die weibliche Brut des Tieres das biologisch aktive Fibrinogen in Milch produziert.

10. Ein Verfahren zum Erzeugen eines transgenen nicht menschlichen Tiers nach einem der Ansprüche 1 bis 9, wobei das Verfahren ein Bereitstellen einer ersten heterologen DNA-Sequenz, die eine Polypeptidkette einer Fibrinogen Aα Untereinheit kodiert, eine zweite heterologe DNA-Sequenz, die eine Polypeptidkette einer Fibrinogen Bβ Untereinheit kodiert und eine dritte heterologe DNA-Sequenz, die ein Polypeptid einer Fibrinogen γ Untereinheit kodiert, ein Einführen der ersten, zweiten und dritten heterologen DNA-Sequenz in das Genom des Säugetiers umfasst.

11. Ein Verfahren nach Anspruch 10, wobei die erste, zweite und dritte DNA-Sequenz in eine befruchtete Eizelle eines nicht menschlichen Säugetiers eingeführt wird und die Eizelle sich in ein vollentwickeltes transgenes Tier entwikkeln kann.

12. Ein Verfahren nach einem der Ansprüche 10 oder 11, ferner umfassend, dass das vollentwickelte transgene Tier Nachkommen erzeugen kann.

13. Ein Verfahren nach einem der Ansprüche 10 oder 11, wobei der Einführungsschritt ein Koinjizieren der ersten, zweiten und dritten heterologen DNA-Sequenz in den Pronukleus der befruchteten Eizelle umfasst.

14. Ein Verfahren zum Erzeugen eines heterologen biologisch aktiven Fibrinogens, das die folgenden Schritte umfasst:
a) Bereitstellen eines transgenen nicht menschlichen Säugetiers nach einem der Ansprüche 1 bis 9,
b) Sammeln von Milch dieses Tiers, und
c) Isolieren oder Reinigen des exprimierten biologisch aktiven Fibrinogenproteins von der Milch.

15. Ein Verfahren nach Anspruch 14, wobei das transgene Tier der Nachkömmling ist, der durch das Verfahren nach Patentanspruch 11 bereitgestellt wird.

16. Ein Verfahren nach Anspruch 14, wobei das transgene Tier durch ein Verfahren bereitgestellt wird mit:
Einführen einer ersten, zweiten und dritten DNA-Sequenz, wie in einem der Ansprüche 1 bis 4 definiert, in eine befruchtete Eizelle eines nicht menschlichen Säugetiers;
Ermöglichen, dass diese Eizelle sich in ein vollentwickeltes transgenes Tier entwickelt;
Ermöglichen, dass dieses Tier weibliche Nachkommen erzeugt, die die erste, zweite und dritte DNA-Sequenzen exprimieren und Milch produzieren, die das biologisch aktive Fibrinogen enthält.

17. Das Tier oder Verfahren nach einem der Ansprüche 1 bis 16, wobei die erste, zweite und dritte DNA-Sequenz jeweils durchführbar mit cis-aktiven Ausführungssequenzen verbunden sind, die zum Ausdruck in der Brustdrüse und Sekretion in Milch erforderlich sind.

18. Das Tier oder Verfahren nach einem der Ansprüche 1 bis 16, wobei die erste, zweite und dritte DNA-Sequenz jeweils durchführbar verbunden ist mit Ausführungssequenzen, die zu deren Ausdruck in der Brustdrüse des Tiers notwendig sind.

19. Das Tier oder Verfahren nach einem der Ansprüche 1 bis 18, wobei die erste, zweite und dritte DNA-Sequenz ferner jeweils durchführbar mit Signalsequenzen verbunden sind, die eine Sekretion von Proteinen in Milch anweisen.

20. Das Tier oder Verfahren nach einem der Ansprüche 1 bis 19, wobei die Ausführungssequenz einen Promoter umfasst, der ausgewählt ist aus der Gruppe bestehend aus einem Molkesäureproteinpromoter, einem Kaseinpromoter, einem β-Lactoglobulinpromoter oder einem α-Lactalbuminpromoter.

21. Das Tier oder Verfahren nach einem der Ansprüche 2 bis 20, wobei die Ausführungssequenz ein Molkesäureproteinpromoter ist.

22. Ein Tier oder Verfahren nach einem der Ansprüche 1 bis 21, wobei das Fibrinogen ein menschliches Fibrinogen ist.

23. Ein Tier oder Verfahren nach einem der Ansprüche 1 bis 22, wobei das transgene nicht menschliche Säugetier ausgewählt ist aus der Gruppe bestehend aus einem Nagetier, einem Kaninchen, einem Schwein, einem Schaf, einer Ziege, einer Kuh und einem Pferd.

24. Das Tier oder Verfahren nach Anspruch 23, wobei das transgene nicht menschliche Säugetier eine Kuh ist.

25. Das Tier oder Verfahren nach einem der Ansprüche 1 bis 24, wobei die erste, zweite und dritte DNA-Sequenz genomische DNA umfasst.

26. Das Tier oder Verfahren nach einem der Ansprüche 1 bis 24, wobei die erste, zweite und dritte DNA-Sequenz cDNA umfasst.

27. Ein nicht menschliches transgenes Säugetier, das durch das Verfahren nach einem der Ansprüche 10 bis 13 oder 17 bis 26 erzeugt wurde, wobei das Tier Milch produziert, die biologisch aktives Fibrinogen enthält.

28. Milch erhalten von dem transgenen nicht menschlichen Säugetier von einem der Ansprüche 1 bis 9, die biologisch aktives Fibrinogen enthält.

## Revendications

1. Animal transgénique mammifère non humain qui a intégré de façon stable dans son génome une première séquence d'ADN hétérologue qui code une chaîne polypeptide sous-unité de fibrinogène Aα, une deuxième séquence d'ADN hétérologue qui code une chaîne polypeptide sous-unité de fibrinogène Bβ et une troisième séquence d'ADN hétérologue qui code une chaîne polypeptide sous-unité de fibrinogène γ, ledit animal ou la progéniture femelle dudit animal étant capable d'exprimer le fibrinogène biologiquement actif dans les cellules de glande mammaire et de sécréter ledit fibrinogène dans le lait dudit animal.

2. Animal transgénique mammifère non humain selon la revendication 1, chez lequel lesdites première, deuxième et troisième séquences d'ADN sont liées de façon opérationnelle aux séquences régulatrices qui dirigent l'expression desdites première, deuxième et troisième séquences d'ADN dans les cellules de glande mammaire, et la sécrétion de fibrinogène biologiquement actif dans le lait.

3. Animal transgénique mammifère non humain selon la revendication 2, chez lequel lesdites première, deuxième et troisième séquences d'ADN sont liées de façon opérationnelle aux séquences régulatrices et signal qui dirigent l'expression desdites première, deuxième et troisième séquences d'ADN dans les cellules de glande mammaire, et la sécrétion de fibrinogène biologiquement actif dans le lait.

4. Animal transgénique mammifère non humain qui a intégré de façon stable dans son génome une première séquence d'ADN hétérologue qui code une chaîne polypeptide sous-unité de fibrinogène Aα, une deuxième séquence d'ADN hétérologue qui code une chaîne polypeptide sous-unité de fibrinogène Bβ et une troisième séquence d'ADN hétérologue qui code une chaîne polypeptide sous-unité de fibrinogène γ, lesdites première, deuxième et troisième séquences d'ADN étant liées de façon opérationnelle aux séquences régulatrices qui dirigent l'expression desdites première, deuxième et troisième séquences d'ADN dans les cellules de glande mammaire, et la sécrétion de fibrinogène biologiquement actif dans le lait.

5. Animal transgénique mammifère non humain qui est le descendant d'un animal transgénique mammifère non humain tel que revendiqué dans l'une quelconque des revendications 1 à 4 et qui est capable d'exprimer du fibrinogène biologiquement actif dans les cellules de glande mammaire, et de sécréter ledit fibrinogène dans le lait dudit animal.

6. Animal transgénique mammifère non humain selon l'une quelconque des revendications 1 à 5, chez lequel ledit animal est capable de transférer ou de transmettre lesdites première, deuxième et troisième séquences d'ADN à sa descendance sous la forme de transgènes intégrés de façon stable dans le génome de ladite descendance.

7. Animal transgénique mammifère non humain selon l'une quelconque des revendications 1 à 6, chez lequel ledit animal est une femelle.

8. Animal transgénique mammifère non humain selon l'une quelconque des revendications 1 à 6, chez lequel ledit animal est un mâle.

9. Animal transgénique mammifère non humain selon l'une quelconque des revendications 1 à 8, chez lequel la progéniture femelle dudit animal produit ledit fibrinogène biologiquement actif dans le lait.

10. Procédé de production d'un animal transgénique mammifère non humain tel que défini dans l'une quelconque des revendications 1 à 9, ledit procédé comprenant l'apport d'une première séquence d'ADN hétérologue qui code une chaîne polypeptide sous-unité de fibrinogène Aα, d'une deuxième séquence d'ADN hétérologue qui code une chaîne polypeptide sous-unité de fibrinogène Bβ et d'une troisième séquence d'ADN hétérologue qui code une chaîne polypeptide sous-unité de fibrinogène γ, et l'introduction desdites première, deuxième et troisième séquences d'ADN hétérologue dans le génome dudit animal mammifère.

11. Procédé selon la revendication 10, dans lequel lesdites première, deuxième et troisième séquences d'ADN sont introduites dans un ovule fécondé d'un animal mammifère non humain, et ledit ovule est laissé se développer en un animal transgénique adulte.

12. Procédé selon la revendication 10 ou la revendication 11, qui comprend en outre la possibilité pour ledit animal transgénique adulte de produire une descendance.

13. Procédé selon la revendication 10 ou la revendication 11, dans lequel ladite étape d'introduction comprend la co-injection desdites première, deuxième et troisième séquences d'ADN hétérologue dans le pronucleus d'un ovule fécondé.

14. Procédé de production d'un fibrogène hétérologue biologiquement actif, qui comprend les étapes :
a) de fourniture d'un animal transgénique mammifère non humain selon l'une quelconque des revendications 1 à 9 ;
b) de collecte du lait dudit animal ; et
c) d'isolement ou de purification de ladite protéine de fibrogène biologiquement actif vis-à-vis dudit lait.

15. Procédé selon la revendication 14, dans lequel ledit animal transgénique est la descendance apportée par le procédé de la revendication 11.

16. Procédé selon la revendication 14, dans lequel ledit animal transgénique est obtenu par un procédé qui comprend :
l'introduction des première, deuxième et troisième séquences d'ADN, telles que définies dans n'importe laquelle des revendications 1 à 4, dans un ovule fécondé d'un animal mammifère non humain ;
la possibilité dudit ovule à se développer en un animal transgénique adulte ;
la possibilité pour ledit animal de produire une descendance femelle qui exprime les première, deuxième et troisième séquences d'ADN et de produire du lait qui contient du fibrinogène biologiquement actif.

17. Animal ou procédé selon l'une quelconque des revendications 1 à 16, dans lequel chacune desdites première, deuxième et troisième séquences d'ADN est liée de façon opérationnelle aux séquences régulatrices agissant en cis, nécessaires pour l'expression dans la glande mammaire et la sécrétion dans le lait.

18. Animal ou procédé selon l'une quelconque des revendications 1 à 16, dans lequel chacune desdites première, deuxième et troisième séquences d'ADN est liée de façon opérationnelle aux séquences régulatrices nécessaires pour leur expression dans la glande mammaire dudit animal.

19. Animal ou procédé selon l'une quelconque des revendications 1 à 18, dans lequel chacune desdites première, deuxième et troisième séquences d'ADN est en outre liée de façon opérationnelle aux séquences signal qui dirigent la sécrétion des protéines dans le lait.

20. Animal ou procédé selon l'une quelconque des revendications 1 à 19, dans lequel ladite séquence régulatrice comprend un promoteur choisi dans le groupe constitué d'un promoteur de protéine d'acide de petit-lait, un promoteur de caséine, un promoteur de β-lactoglobuline, ou un promoteur d'α-lactalbumine.

21. Animal ou procédé selon l'une quelconque des revendications 2 à 20, dans lequel ladite séquence régulatrice est un promoteur de protéine d'acide de petit-lait.

22. Animal ou procédé selon l'une quelconque des revendications 1 à 21, dans lequel ledit fibrinogène est du fibrinogène humain.

23. Animal ou procédé selon l'une quelconque des revendications 1 à 22, dans lequel ledit animal transgénique mammifère non humain est choisi dans le groupe constitué d'un rongeur, d'un lapin, d'un porc, d'un mouton, d'une chèvre, d'une vache et d'un cheval.

24. Animal ou procédé selon la revendication 23, dans lequel ledit animal transgénique mammifère non humain est une vache.

25. Animal ou procédé selon l'une quelconque des revendications 1 à 24, dans lequel lesdites première, deuxième et troisième séquences d'ADN comprennent de l'ADN génomique.

26. Animal ou procédé selon l'une quelconque des revendications 1 à 24, dans lequel lesdites première, deuxième et troisième séquences d'ADN comprennent du cADN.

27. Animal transgénique mammifère non humain obtenu par le procédé selon l'une quelconque des revendications 10 à 13 ou 17 à 26, chez lequel ledit animal produit du lait contenant du fibrinogène biologiquement actif.

28. Lait obtenu à partir de l'animal transgénique mammifère non humain selon l'une quelconque des revendications 1 à 9, qui contient du fibrinogène biologiquement actif.
